# EUROPÄISCHE PATENTSCHRIFT

(11) **EP 4 408 346 B1**
(45) Veröffentlichungstag und Bekanntmachung des Hinweises auf die Patenterteilung: **10.12.2025**
(21) Anmeldenummer: 22797682.6
(22) Anmeldetag: 26.09.2022
(51) Int. Cl.: A61F 2/16

(54) **VERFAHREN ZUR VERLAGERUNG VON HAPTIKEN EINER INTRAOKULARLINSE IN EINEM INJEKTOR UND INJEKTOR HIERFÜR**
METHOD FOR DISPLACING HAPTICS OF AN INTRAOCULAR LENS IN AN INJECTOR AND INJECTOR FOR THAT
PROCÉDÉ DE DÉPLACEMENT DE L'HAPTIQUE D'UNE LENTILLE INTRAOCULAIRE DANS UN INJECTEUR ET INJECTEUR À CET EFFET

(30) Priorität: 29.09.2021 DE 102021125297
(43) Veröffentlichungstag der Anmeldung: 07.08.2024
(73) Patentinhaber: Carl Zeiss Meditec AG, 07745 Jena (DE)
(72) Erfinder: DAMM, Niklas, 73447 Oberkochen (DE); SRBINOSKA, Hristina, 73447 Oberkochen (DE)
(74) Vertreter: PATERIS Patentanwälte PartmbB
(86) Internationale Anmeldenummer: PCT/EP2022/076701
(87) Internationale Veröffentlichungsnummer: WO 2023/052302

(56) Entgegenhaltungen:
- EP-A1- 0 175 972
- US-A- 4 298 996
- US-A1- 2019 192 283

## Beschreibung

Die Erfindung betrifft eine Intraokularlinse und einen Injektor mit der Intraokularlinse.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges beispielsweise mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Spitze passt. Die Spitze wird durch den Schnitt in den Kapselsack eingeführt und die gefaltete Intraokularlinse wird mittels des Injektors durch die Spitze in den Kapselsack geschoben, in dem die Intraokularlinse sich entfaltet und somit die ursprüngliche Linse ersetzt. Während die Intraokularlinse gefaltet wird oder während die gefaltete Intraokularlinse in den Kapselsack geschoben wird, kann es zu Komplikationen kommen. Die Komplikationen können beispielsweise dazu führen, dass sich die Intraokularlinse nicht vollständig in dem Kapselsack entfaltet oder dass die Intraokularlinse sogar beschädigt wird.

US 4 969 897 offenbart eine Intraokularlinse mit einem flexiblen Positionierungsschenkel, der durch Zusammenwirken zwischen dem Schenkel und Mitteln am Linsenkörper lösbar in einer eingezogenen Position gehalten wird. US 5 593 437 A offenbart eine Vorrichtung zum Einstellen der Position eines Brennpunktes eines intraokularen Implantats, das teilweise mit einem magnetischen Material versehen ist. US 4 298 996 A offenbart eine Linse, die nach Entfernung der Augenlinse in das Auge implantiert wird. US 2019/0192283 A1 offenbart ein Injektionssystem für eine ophthalmische Operation.

Aufgabe der Erfindung ist es daher, ein Verfahren mit einer Intraokularlinse und einen Injektor mit der Intraokularlinse zu schaffen, mit denen eine Wahrscheinlichkeit gering ist, mit der Komplikationen bei einer Benutzung des Injektors auftreten.

Bei einem ersten erfindungsgemäßen Verfahren weist eine erste erfindungsgemäße Intraokularlinse einen Optikkörper mit einer optischen Achse, eine erste Haptik und eine zweite Haptik auf. Die erste Haptik ist gekrümmt ausgeführt und weist ein erstes Haptiklängsende, das dem Optikkörper abgewandt angeordnet ist, und einen ersten Permanentmagneten auf. Die zweite Haptik ist gekrümmt ausgeführt und weist ein zweites Haptiklängsende, das dem Optikkörper abgewandt angeordnet ist, und einen zweiten Permanentmagneten auf. Die erste Haptik hat einen Beabstandungszustand, in dem das erste Haptiklängsende in einem ersten Radialabstand zu dem Optikkörper in Richtung zu der optischen Achse hin angeordnet ist, und einen Annäherungszustand, in dem das erste Haptiklängsende auf den Optikkörper verlagert ist, wodurch in einer Axialrichtung bezüglich der optischen Achse gesehen das erste Haptiklängsende in dem Bereich des Optikkörpers angeordnet ist. Die zweite Haptik hat einen Beabstandungszustand, in dem das zweite Haptiklängsende in einem zweiten Radialabstand zu dem Optikkörper in Richtung zu der optischen Achse hin angeordnet ist, und einen Annäherungszustand, in dem das zweite Haptiklängsende auf den Optikkörper verlagert ist, wodurch in der Axialrichtung gesehen das zweite Haptiklängsende in dem Bereich des Optikkörpers angeordnet ist. Die erste Haptik wird durch ein Beaufschlagen des ersten Permanentmagneten mit einem ersten Magnetfeld von dem Beabstandungszustand in den Annäherungszustand verlagert und die zweite Haptik wird durch ein Beaufschlagen des zweiten Permanentmagneten mit einem zweiten Magnetfeld von dem Beabstandungszustand in den Annäherungszustand verlagert.

Bei einem zweiten erfindungsgemäßen Verfahren weist eine zweite erfindungsgemäße Intraokularlinse einen Optikkörper mit einer optischen Achse, eine erste Haptik und eine zweite Haptik auf. Die erste Haptik ist gekrümmt ausgeführt und weist ein erstes Haptiklängsende, das dem Optikkörper abgewandt angeordnet ist, und ein erstes magnetisierbares Bauteil auf. Die zweite Haptik ist gekrümmt ausgeführt und weist ein zweites Haptiklängsende, das dem Optikkörper abgewandt angeordnet ist, und ein zweites magnetisierbares Bauteil auf. Die erste Haptik hat einen Beabstandungszustand, in dem das erste Haptiklängsende in einem ersten Radialabstand zu dem Optikkörper in Richtung zu der optischen Achse hin angeordnet ist, und einen Annäherungszustand, in dem das erste Haptiklängsende auf den Optikkörper verlagert ist, wodurch in einer Axialrichtung bezüglich der optischen Achse gesehen das erste Haptiklängsende in dem Bereich des Optikkörpers angeordnet ist. Die zweite Haptik hat einen Beabstandungszustand, in dem das zweite Haptiklängsende in einem zweiten Radialabstand zu dem Optikkörper in Richtung zu der optischen Achse hin angeordnet ist, und einen Annäherungszustand, in dem das zweite Haptiklängsende auf den Optikkörper verlagert ist, wodurch in der Axialrichtung gesehen das zweite Haptiklängsende in dem Bereich des Optikkörpers angeordnet ist. Die erste Haptik wird durch ein Beaufschlagen des ersten magnetisierbaren Bauteils mit einem ersten Magnetfeld von dem Beabstandungszustand in den Annäherungszustand verlagert und die zweite Haptik wird durch ein Beaufschlagen des zweiten magnetisierbaren Bauteils mit einem zweiten Magnetfeld von dem Beabstandungszustand in den Annäherungszustand verlagert.

Der erfindungsgemäße Injektor weist die Intraokularlinse, die in dem Injektor angeordnet ist, und einen ersten Elektromagneten auf, der eingerichtet ist, die erste Haptik von dem Beabstandungszustand in den Annäherungszustand zu verlagern, wobei der Injektor eine Injektorspitze, eine Injektoröffnung, einen Faltkeil, der eingerichtet ist, via die Injektoröffnung innerhalb des Injektors zu gelangen und dabei die Intraokularlinse zu falten, eine Injektorlängsachse und einen in Richtung der Injektorlängsachse verlagerbaren Kolben aufweist, der eingerichtet die Intraokularlinse via die Injektorspitze aus dem Injektor heraus zu verlagern.

Durch das Beaufschlagen der ersten Haptik und der zweiten Haptik mit dem jeweiligen Magnetfeld und dadurch, dass die erste Haptik und die zweite Haptik jeweils gekrümmt ausgeführt sind, können die erste Haptik und die zweite Haptik durch ein Verbiegen in ihren jeweiligen Annäherungszustand gebracht werden. Anschließend kann die Intraokularlinse gefaltet werden, indem der Faltkeil innerhalb des Injektors gebracht wird. Die erste Haptik und die zweite Haptik sind nach dem Falten innerhalb des Optikkörpers angeordnet. Dadurch kann ein fehlerarmes und reproduzierbares Einbringen der Intraokularlinse in den Kapselsack eines Auges und ein fehlerarmes und reproduzierbares Entfalten der Intraokularlinse in dem Kapselsack erreicht werden. Durch das Verwenden des magnetisierbaren ersten Bauteils und des magnetisierbaren zweiten Bauteils, die nur magnetisch sind, wenn sie von einem Magnetfeld beaufschlagt werden, kann das Auftreten von Problemen bei einer Diagnostik, insbesondere eine Magnetresonanztomographie, eines die Intraokularlinse tragenden Menschen vorteilhaft verringert werden.

Der erste Elektromagnet kann eingerichtet sein, sowohl die erste Haptik als auch die zweite Haptik von dem Beabstandungszustand in den Annäherungszustand zu verlagern. Alternativ ist denkbar, dass ein zweiter Elektromagnet vorgesehen ist, der eingerichtet ist, die zweite Haptik von dem Beabstandungszustand in den Annäherungszustand zu verlagern. Der erste Elektromagnet und/oder der zweite Elektromagnet können in ihrem eingeschalteten Zustand entlang der Injektorlängsachse polarisiert sein. Durch ein Einschalten des ersten Elektromagneten und optional des zweiten Elektromagneten kann ein Bediener des Injektors die erste Haptik und die zweite Haptik in den Annäherungszustand bringen. Über die Stärke des Magnetfeldes des ersten Elektromagneten und optional die Stärke des Magnetfeldes des zweiten Elektromagneten kann eingestellt werden, wie weit die erste Haptik und die zweite Haptik verlagern.

Für die erste erfindungsgemäße Intraokularlinse und die zweite erfindungsgemäße Intraokularlinse gilt, dass die erste Haptik und/oder die zweite Haptik bevorzugt C-förmig oder J-förmig sind.

Der erste Permanentmagnet ist bevorzugt in einem Haptikbereich der ersten Haptik angeordnet, der sich ausgehend von dem ersten Haptiklängsende bis zu maximal 50 %, insbesondere maximal 20 % oder maximal 10 %, der Gesamtlänge der ersten Haptik erstreckt und/oder der zweite Permanentmagnet ist bevorzugt in einem Haptikbereich der zweiten Haptik angeordnet, der sich ausgehend von dem zweiten Haptiklängsende bis zu maximal 50 %, insbesondere maximal 20 % oder maximal 10 %, der Gesamtlänge der zweiten Haptik erstreckt. Dadurch ist ein Hebelarm bei dem Verlagern der jeweiligen Haptik besonders lang und somit kann die jeweilige Haptik besonders einfach in den Annäherungszustand verlagert werden.

Es ist bevorzugt, dass eine Mitte zwischen einem Nordpol und einem Südpol des ersten Permanentmagneten sich im Wesentlichen entlang einer Längsrichtung der ersten Haptik erstreckt und eine Mitte zwischen einem Nordpol und/oder einem Südpol des zweiten Permanentmagneten sich im Wesentlichen entlang einer Längsrichtung der zweiten Haptik erstreckt. Dadurch sind der erste Permanentmagnet und/oder der zweite Permanentmagnet im Wesentlichen in der Richtung polarisiert, in der die jeweilige Haptik von dem Beabstandungszustand in den Annäherungszustand zu verlagern ist.

Es ist bevorzugt, dass der erste Permanentmagnet die Form eines ersten Stabs hat, der eine Längsrichtung hat, die sich im Wesentlichen entlang einer Längsrichtung der ersten Haptik erstreckt, und/oder der zweite Permanentmagnet die Form eines zweiten Stabs hat, der eine Längsrichtung hat, die sich im Wesentlichen entlang einer Längsrichtung der zweiten Haptik erstreckt. Der erste Stab und/oder der zweite Stab können beispielsweise einen kreisförmigen, rechteckigen oder quadratischen Querschnitt haben.

Der erste Permanentmagnet weist bevorzugt eine erste Mehrzahl an Einzelmagneten auf, die beabstandet voneinander angeordnet sind, und/oder der zweite Permanentmagnet weist bevorzugt eine zweite Mehrzahl an Einzelmagneten auf, die beabstandet voneinander angeordnet sind. Die Einzelmagneten des ersten Permanentmagneten und/oder die Einzelmagneten des zweiten Permanentmagneten können beispielsweise die Form einer Kugel haben. Alternativ ist denkbar, dass die Einzelmagneten des ersten Permanentmagneten und/oder die Einzelmagneten des zweiten Permanentmagneten eine asphärische Form haben.

Es ist bevorzugt, dass der erste Permanentmagnet als eine erste Beschichtung auf die erste Haptik aufgebracht ist und/oder der zweite Permanentmagnet als eine zweite Beschichtung auf die zweite Haptik aufgebracht ist.

Der erste Permanentmagnet weist bevorzugt einen ersten Faden oder mehrere erste Fäden auf und/oder der zweite Permanentmagnet weist bevorzugt einen zweiten Fäden und/oder mehrere zweite Fäden auf. Die Fäden können so ausgeführt sein, dass die zugehörige Haptik versteift. Die Fäden können nach dem Einsetzen der Intraokularlinse in den Kapselsack eines Auges entfernt werden und insbesondere durch Polymerfäden ersetzt werden.

Es ist bevorzugt, dass die erste Haptik eine erste Ummantelung aufweist, die nicht magnetisch und nicht magnetisierbar ist und den ersten Permanentmagneten vollständig ummantelt und/oder dass die zweite Haptik bevorzugt eine zweite Ummantelung aufweist, die nicht magnetisch und nicht magnetisierbar ist und den zweiten Permanentmagneten vollständig ummantelt. Dadurch kann vorteilhaft ein Kontakt des ersten Permanentmagneten mit dem ersten Elektromagneten und/oder des zweiten Permanentmagneten mit dem zweiten Elektromagneten unterbunden werden.

Die erste Haptik weist bevorzugt eine erste Pore auf, in die der erste Permanentmagnet in Form von Nanopartikeln eingebracht ist und/oder die zweite Haptik weist bevorzugt eine zweite Pore auf, in die der zweite Permanentmagnet in Form von Nanopartikeln eingebracht ist. Die Nanopartikel können nach einem Einsetzen der Intraokularlinse in den Kapselsack eines Auges von einer Augenflüssigkeit aus den Poren herausgeschwemmt werden und von dem Trabekelwerk des Auges aus der Augenflüssigkeit herausgeschwemmt werden. Die Poren können beispielsweise mittels eines Lasers in die jeweilige Haptik eingebracht sein. Es ist auch denkbar, eine Mehrzahl der ersten Poren in der ersten Haptik vorzusehen und/oder eine Mehrzahl der zweiten Poren in der zweiten Haptik vorzusehen.

Der erste Permanentmagnet und/oder der zweite Permanentmagnet können resorbierbar ausgeführt sein. Dazu können der erste Permanentmagnet und/oder der zweite Permanentmagnet jeweils in Form von Nanopartikeln vorliegen, die von einem resorbierbaren Polymer, insbesondere PLGA (Polylactid-co-Glycolid), umhüllt sind. Diese Nanopartikel können in die erste Pore und/oder die zweite Pore eingebracht sein und/oder als die erste Beschichtung und/oder als die zweite Beschichtung aufgebracht sein.

Das erste Bauteil ist bevorzugt in einem Haptikbereich der ersten Haptik angeordnet, der sich ausgehend von dem ersten Haptiklängsende bis zu maximal 50 %, insbesondere maximal 20 % oder maximal 10 %, der Gesamtlänge der ersten Haptik erstreckt und/oder das zweite Bauteil ist bevorzugt in einem Haptikbereich der zweiten Haptik angeordnet, der sich ausgehend von dem zweiten Haptiklängsende bis zu maximal 50 %, insbesondere maximal 20 % oder maximal 10 %, der Gesamtlänge der zweiten Haptik erstreckt.

Es ist bevorzugt, dass eine Mitte zwischen einem Nordpol und einem Südpol des magnetisierten ersten Bauteils sich im Wesentlichen entlang einer Längsrichtung der ersten Haptik erstreckt und eine Mitte zwischen einem Nordpol und/oder einem Südpol des magnetisierten zweiten Bauteils sich im Wesentlichen entlang einer Längsrichtung der zweiten Haptik erstreckt.

Es ist bevorzugt, dass das erste Bauteil die Form eines ersten Stabs hat, der eine Längsrichtung hat, die sich im Wesentlichen entlang einer Längsrichtung der ersten Haptik erstreckt, und/oder das zweite Bauteil die Form eines zweiten Stabs hat, der eine Längsrichtung hat, die sich im Wesentlichen entlang einer Längsrichtung der zweiten Haptik erstreckt. Der erste Stab und/oder der zweite Stab können beispielsweise einen kreisförmigen, rechteckigen oder quadratischen Querschnitt haben.

Das erste Bauteil weist bevorzugt eine erste Mehrzahl an Partikeln auf, die beabstandet voneinander angeordnet sind, und/oder das zweite Bauteil weist bevorzugt eine zweite Mehrzahl an Partikeln auf, die beabstandet voneinander angeordnet sind. Die Partikel des ersten Bauteils und/oder die Partikel des zweiten Bauteils können beispielsweise die Form einer Kugel haben. Alternativ ist denkbar, dass die Partikel des ersten Bauteils und/oder die Partikel des zweiten Bauteils eine asphärische Form haben.

Es ist bevorzugt, dass das erste Bauteil als eine erste Beschichtung auf die erste Haptik aufgebracht ist und/oder dass das zweite Bauteil als eine zweite Beschichtung auf die zweite Haptik aufgebracht ist.

Das erste Bauteil weist bevorzugt einen ersten Faden oder mehrere erste Fäden auf und/oder das zweite Bauteil weist bevorzugt einen zweiten Fäden und/oder mehrere zweite Fäden auf. Die Fäden können so ausgeführt sein, dass die zugehörige Haptik versteift. Die Fäden können nach dem Einsetzen der Intraokularlinse in den Kapselsack eines Auges entfernt werden und insbesondere durch Polymerfäden ersetzt werden.

Es ist bevorzugt, dass die erste Haptik eine erste Ummantelung aufweist, die nicht magnetisch und nicht magnetisierbar ist und das erste Bauteil vollständig ummantelt und/oder dass die zweite Haptik bevorzugt eine zweite Ummantelung aufweist, die nicht magnetisch und nicht magnetisierbar ist und das zweite Bauteil vollständig ummantelt.

Die erste Haptik weist bevorzugt eine erste Pore auf, in die das erste Bauteil in Form von Nanopartikeln eingebracht ist und/oder die zweite Haptik weist bevorzugt eine zweite Pore auf, in die das zweite Bauteil in Form von Nanopartikeln eingebracht ist. Die Poren können beispielsweise mittels eines Lasers in die jeweilige Haptik eingebracht sein. Es ist auch denkbar, eine Mehrzahl der ersten Poren in der ersten Haptik vorzusehen und/oder eine Mehrzahl der zweiten Poren in der zweiten Haptik vorzusehen.

Das erste Bauteil und/oder das zweite Bauteil können resorbierbar ausgeführt sein. Dazu können das erste Bauteil und/oder das zweite Bauteil jeweils in Form von Nanopartikeln vorliegen, die von einem resorbierbaren Polymer, insbesondere PLGA (Polylactid-co-Glycolid), umhüllt sind. Diese Nanopartikel können in die erste Pore und/oder die zweite Pore eingebracht sein und/oder als die erste Beschichtung und/oder als die zweite Beschichtung aufgebracht sein.

Es ist bevorzugt, dass der Injektor einen ersten Führungskanal und einen dem ersten Führungskanal zugewandt angeordneten zweiten Führungskanal aufweist, wobei in dem ersten Führungskanal ein Teil des Optikkörpers, das Längsende der ersten Haptik und der erste Elektromagnet angeordnet sind und in dem zweiten Führungskanal ein Teil des Optikkörpers und das Längsende der zweiten Haptik angeordnet sind. Dadurch sind das erste Haptiklängsende und das zweite Haptiklängsende vorteilhaft bei ihrem Verlagern geführt.

Der erste Führungskanal und der zweite Führungskanal sind bevorzugt gekrümmt ausgeführt. Dadurch kann ein Verlagern des ersten Haptiklängsendes und des zweiten Haptiklängsendes auf den Optikkörper vereinfacht werden.

Im Folgenden wird anhand der beigefügten schematischen Zeichnungen die Erfindung näher erläutert. Es zeigen
Figur 1 eine Draufsicht auf eine erste Ausführungsform der Intraokularlinse,
Figur 2 eine Draufsicht auf eine zweite Ausführungsform der Intraokularlinse,
Figur 3 eine perspektivische Ansicht auf einen Injektor mit einer Intraokularlinse in einem Beabstandungszustand und
Figur 4 einen Längsschnitt durch den Injektor gemäß Figur 3 mit der Intraokularlinse in einem Annäherungszustand.

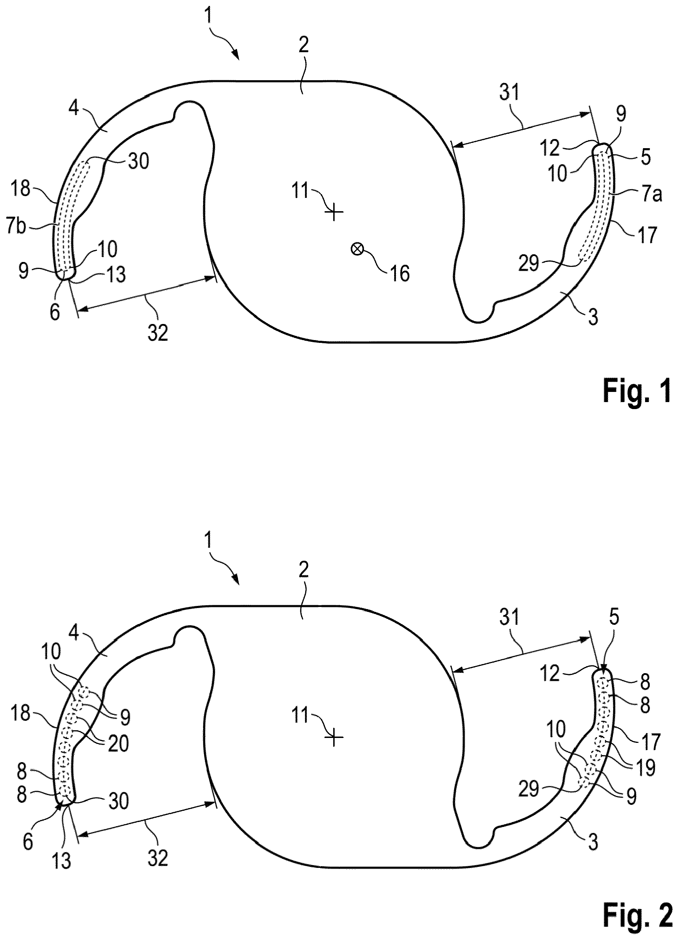

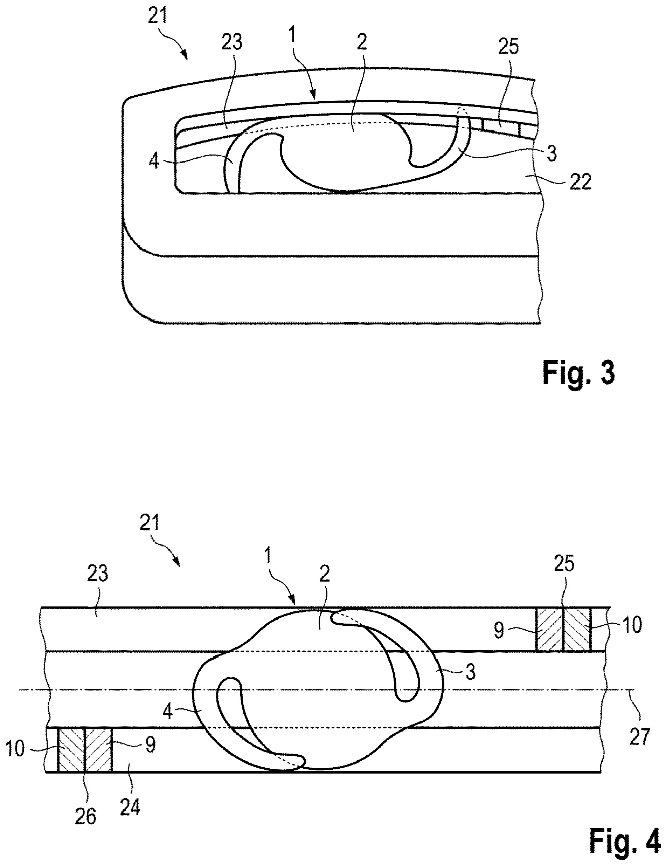

Wie es aus Figuren 1 und 2 ersichtlich ist, weist eine Intraokularlinse 1 einen Optikkörper 2, der eine optische Achse 11 aufweist, eine erste Haptik 3, die an dem Optikkörper 2 befestigt ist, und eine zweite Haptik 4 auf, die an dem Optikkörper 2 befestigt ist. Die erste Haptik 3 ist gekrümmt ausgeführt und weist ein erstes Haptiklängsende 12, das dem Optikkörper 2 abgewandt angeordnet ist, und einen ersten Permanentmagneten 5 auf. Die zweite Haptik 4 ist gekrümmt ausgeführt und weist ein zweites Haptiklängsende 13, das dem Optikkörper 2 abgewandt angeordnet ist, und einen zweiten Permanentmagneten 6 auf. Die erste Haptik 3 hat einen Beabstandungszustand, in dem das erste Haptiklängsende 12 in einem ersten Radialabstand 31 zu dem Optikkörper 2 in Richtung zu der optischen Achse 11 hin angeordnet ist, und einen Annäherungszustand, in dem das erste Haptiklängsende 12 auf den Optikkörper 2 verlagert ist, wodurch in einer Axialrichtung 16 bezüglich der optischen Achse 11 gesehen das erste Haptiklängsende 12 in dem Bereich des Optikkörpers 2 angeordnet ist (siehe Figur 4). Die zweite Haptik 4 hat einen Beabstandungszustand, in dem das zweite Haptiklängsende 13 in einem zweiten Radialabstand 32 zu dem Optikkörper 2 in Richtung zu der optischen Achse 11 hin angeordnet ist, und einen Annäherungszustand, in dem das zweite Haptiklängsende 13 auf den Optikkörper 2 verlagert ist, wodurch in der Axialrichtung 16 gesehen das zweite Haptiklängsende 13 in dem Bereich des Optikkörpers 2 angeordnet ist (siehe Figur 4). Die erste Haptik 3 ist durch ein Beaufschlagen des ersten Permanentmagneten 5 mit einem ersten Magnetfeld von dem Beabstandungszustand in den Annäherungszustand verlagerbar und die zweite Haptik 4 ist durch ein Beaufschlagen des zweiten Permanentmagneten 6 mit einem zweiten Magnetfeld von dem Beabstandungszustand in den Annäherungszustand verlagerbar.

Figur 1 zeigt, dass gemäß der ersten Ausführungsform der Intraokularlinse 1 der erste Permanentmagnet 5 die Form eines ersten Stabs 7a haben kann, der eine Längsrichtung hat, die sich im Wesentlichen entlang einer Längsrichtung der ersten Haptik 3 erstreckt, und der zweite Permanentmagnet 6 die Form eines zweiten Stabs 7b haben kann, der eine Längsrichtung hat, die sich im Wesentlichen entlang einer Längsrichtung der zweiten Haptik 4 erstreckt.

Figur 2 zeigt, dass gemäß der zweiten Ausführungsform der Intraokularlinse 1 der erste Permanentmagnet 5 eine erste Mehrzahl an Einzelmagneten 19 aufweisen kann, die beabstandet voneinander angeordnet sind, und der zweite Permanentmagnet 6 eine zweite Mehrzahl an Einzelmagneten 20 aufweisen kann, die beabstandet voneinander angeordnet sind. Die Einzelmagneten 19 des ersten Permanentmagneten 5 und die Einzelmagneten 20 des zweiten Permanentmagneten 6 können, wie in Figur 2 dargestellt, die Form einer Kugel 8 haben.

Wie es aus Figuren 1 und 2 ersichtlich ist, kann der erste Permanentmagnet 5 in einem Haptikbereich der ersten Haptik 3 angeordnet sein, der sich ausgehend von dem ersten Haptiklängsende 12 bis zu maximal 50 %, insbesondere maximal 20 % oder maximal 10 %, der Gesamtlänge der ersten Haptik 3 erstreckt und der zweite Permanentmagnet 6 kann in einem Haptikbereich der zweiten Haptik 4 angeordnet sein, der sich ausgehend von dem zweiten Haptiklängsende 13 bis zu maximal 50 %, insbesondere maximal 20 % oder maximal 10 %, der Gesamtlänge der zweiten Haptik 4 erstreckt.

Zudem zeigen Figuren 1 und 2, dass eine Mitte 29 zwischen einem Nordpol 9 und einem Südpol 10 des ersten Permanentmagneten 5 sich im Wesentlichen entlang einer Längsrichtung der ersten Haptik 3 erstrecken kann und eine Mitte 30 zwischen einem Nordpol 9 und/oder einem Südpol 10 des zweiten Permanentmagneten 6 sich im Wesentlichen entlang einer Längsrichtung der zweiten Haptik 4 erstrecken kann.

Die erste Haptik 3 kann eine erste Ummantelung 17 aufweisen, die nicht magnetisch und nicht magnetisierbar ist und den ersten Permanentmagneten 5 vollständig ummantelt und die zweite Haptik 4 kann eine zweite Ummantelung 18 aufweisen, die nicht magnetisch und nicht magnetisierbar ist und den zweiten Permanentmagneten 6 vollständig ummantelt, vergleiche Figuren 1 und 2.

Wie es aus Figuren 3 und 4 ersichtlich ist, weist ein Injektor 21 die Intraokularlinse 1, die in dem Injektor 21 angeordnet ist, einen ersten Elektromagneten 25, der eingerichtet ist, die erste Haptik 3 von dem Beabstandungszustand in den Annäherungszustand zu verlagern, und einen zweiten Elektromagneten 26 auf, der eingerichtet, die zweite Haptik 4 von dem Beabstandungszustand 25 in den Annäherungszustand zu verlagern. Der Injektor 21 weist zudem eine Injektorspitze (nicht in Figuren 3 und 4 dargestellt), eine Injektoröffnung 22, einen Faltkeil (nicht in Figuren 3 und 4 dargestellt), der eingerichtet ist, via die Injektoröffnung 22 innerhalb des Injektors 21 zu gelangen und dabei die Intraokularlinse 1 zu falten, eine Injektorlängsachse 27 und einen in Richtung der Injektorlängsachse 27 verlagerbaren Kolben (nicht in Figuren 3 und 4 dargestellt) auf, der eingerichtet die Intraokularlinse 1 via die Injektorspitze aus dem Injektor 21 heraus zu verlagern. Figur 4 zeigt, dass die erste Haptik 3 in Richtung der Injektorlängsachse 27 zwischen dem Optikkörper 2 und dem ersten Elektromagneten 25 angeordnet sein kann und dass die zweite Haptik 4 in Richtung der Injektorlängsachse 27 zwischen dem Optikkörper 2 und dem zweiten Elektromagneten 26 angeordnet sein kann. Der erste Elektromagnet 25 kann gegenpolig zu dem ersten Permanentmagneten 5 angeordnet sein und der zweite Elektromagnet 26 kann gegenpolig zu dem zweiten Permanentmagneten 6 angeordnet sein. Zudem können der erste Elektromagnet 25 und der zweite Elektromagnet 26 in Richtung der Injektorlängsachse 27 polarisiert sein.

Figur 3 zeigt, dass der Injektor 21 einen ersten Führungskanal 23 und einen dem ersten Führungskanal 23 zugewandt angeordneten zweiten Führungskanal 24 aufweisen kann, wobei in dem ersten Führungskanal 23 ein Teil des Optikkörpers 2, das Längsende 12 der ersten Haptik 3 und der erste Elektromagnet 25 angeordnet sind und in dem zweiten Führungskanal 24 ein Teil des Optikkörpers 2 und das Längsende 13 der zweiten Haptik 4 angeordnet sind. Der erste Führungskanal 23 und der zweite Führungskanal 24 können gekrümmt ausgeführt sein.

### Bezugszeichenliste

- 1: Intraokularlinse
- 2: Optikkörper
- 3: erste Haptik
- 4: zweite Haptik
- 5: erster Permanentmagnet
- 6: zweiter Permanentmagnet
- 7a: Stab
- 7b: Stab
- 8: Kugel
- 9: Nordpol
- 10: Südpol
- 11: optische Achse
- 12: erstes Haptiklängsende
- 13: zweites Haptiklängsende
- 16: Axialrichtung
- 17: erste Ummantelung
- 18: zweite Ummantelung
- 19: Einzelmagnet
- 20: Einzelmagnet
- 21: Injektor
- 22: Injektoröffnung
- 23: erster Führungskanal
- 24: zweiter Führungskanal
- 25: erster Elektromagnet
- 26: zweiter Elektromagnet
- 27: Injektorlängsachse
- 29: Mitte des ersten Permanentmagneten
- 30: Mitte des zweiten Permanentmagneten
- 31: erster Radialabstand
- 32: zweiter Radialabstand

## Patentansprüche

1. Verfahren zur Verlagerung von Haptiken einer Intraokularlinse, bei dem die Intraokularlinse (1) in einem Injektor (21) angeordnet ist, wobei die Intraokularlinse (1) einen Optikkörper (2), der eine optische Achse (11) aufweist, eine erste Haptik (3), die gekrümmt ausgeführt ist und ein erstes Haptiklängsende (12) aufweist, das dem Optikkörper (2) abgewandt angeordnet ist, und eine zweite Haptik (4) aufweist, die gekrümmt ausgeführt ist und ein zweites Haptiklängsende (13) aufweist, das dem Optikkörper (2) abgewandt angeordnet ist, wobei die erste Haptik (3) einen Beabstandungszustand, in dem das erste Haptiklängsende (12) in einem ersten Radialabstand (31) zu dem Optikkörper (2) in Richtung zu der optischen Achse (11) hin angeordnet ist, und einen Annäherungszustand hat, in dem das erste Haptiklängsende (12) auf den Optikkörper (2) verlagert ist, wodurch in einer Axialrichtung (16) bezüglich der optischen Achse (11) gesehen das erste Haptiklängsende (12) in dem Bereich des Optikkörpers (2) angeordnet ist, wobei die zweite Haptik (4) einen Beabstandungszustand, in dem das zweite Haptiklängsende (13) in einem zweiten Radialabstand (32) zu dem Optikkörper (2) in Richtung zu der optischen Achse (11) hin angeordnet ist, und einen Annäherungszustand hat, in dem das zweite Haptiklängsende (13) auf den Optikkörper (2) verlagert ist, wodurch in der Axialrichtung (16) gesehen das zweite Haptiklängsende (13) in dem Bereich des Optikkörpers (2) angeordnet ist, **dadurch gekennzeichnet, dass** die erste Haptik (3) einen ersten Permanentmagneten (5) aufweist, die zweite Haptik (4) einen zweiten Permanentmagneten (6) aufweist, der Injektor (21) einen ersten Elektromagneten (25) aufweist, der eingerichtet ist, die erste Haptik (3) von dem Beabstandungszustand in den Annäherungszustand zu verlagern, und das Verfahren die Schritte aufweist:
- Verlagern der ersten Haptik (3) durch ein Beaufschlagen des ersten Permanentmagneten (5) mit einem ersten Magnetfeld von dem Beabstandungszustand in den Annäherungszustand;
- Verlagern der zweiten Haptik (4) durch ein Beaufschlagen des zweiten Permanentmagneten (6) mit einem zweiten Magnetfeld von dem Beabstandungszustand in den Annäherungszustand.

2. Verfahren gemäß Anspruch 1, wobei der erste Permanentmagnet (5) in einem Haptikbereich der ersten Haptik (3) angeordnet ist, der sich ausgehend von dem ersten Haptiklängsende (12) bis zu maximal 50 %, insbesondere maximal 20 % oder maximal 10 %, der Gesamtlänge der ersten Haptik (3) erstreckt und/oder wobei der zweite Permanentmagnet (6) in einem Haptikbereich der zweiten Haptik (4) angeordnet ist, der sich ausgehend von dem zweiten Haptiklängsende (13) bis zu maximal 50 %, insbesondere maximal 20 % oder maximal 10 %, der Gesamtlänge der zweiten Haptik (4) erstreckt.

3. Verfahren gemäß Anspruch 1 oder 2, wobei eine Mitte (29) zwischen einem Nordpol (9) und einem Südpol (10) des ersten Permanentmagneten (5) im Wesentlichen entlang einer Längsrichtung der ersten Haptik (3) sich erstreckt und eine Mitte (30) zwischen einem Nordpol (9) und/oder einem Südpol (10) des zweiten Permanentmagneten (6) im Wesentlichen entlang einer Längsrichtung der zweiten Haptik (4) sich erstreckt.

4. Verfahren gemäß einem der Ansprüche 1 bis 3, wobei der erste Permanentmagnet (5) als eine erste Beschichtung auf die erste Haptik (3) aufgebracht ist und/oder der zweite Permanentmagnet (6) als eine zweite Beschichtung auf die zweite Haptik (4) aufgebracht ist.

5. Verfahren gemäß einem der Ansprüche 1 bis 4, wobei die erste Haptik (3) eine erste Ummantelung (16) aufweist, die nicht magnetisch und nicht magnetisierbar ist und den ersten Permanentmagneten (5) vollständig ummantelt und/oder wobei die zweite Haptik (4) eine zweite Ummantelung (17) aufweist, die nicht magnetisch und nicht magnetisierbar ist und den zweiten Permanentmagneten (6) vollständig ummantelt.

6. Verfahren zur Verlagerung von Haptiken einer Intraokularlinse, bei dem die Intraokularlinse (1) in einem Injektor (21) angeordnet ist, wobei die Intraokularlinse (1) einen Optikkörper (2), der eine optische Achse (11) aufweist, eine erste Haptik (3), die gekrümmt ausgeführt ist und ein erstes Haptiklängsende (12) aufweist, das dem Optikkörper (2) abgewandt angeordnet ist, und eine zweite Haptik (4) aufweist, die gekrümmt ausgeführt ist und ein zweites Haptiklängsende (13) aufweist, das dem Optikkörper (2) abgewandt angeordnet ist, wobei die erste Haptik (3) einen Beabstandungszustand, in dem das erste Haptiklängsende (12) in einem ersten Radialabstand (31) zu dem Optikkörper (2) in Richtung zu der optischen Achse (11) hin angeordnet ist, und einen Annäherungszustand hat, in dem das erste Haptiklängsende (12) auf den Optikkörper (2) verlagert ist, wodurch in einer Axialrichtung (16) bezüglich der optischen Achse (11) gesehen das erste Haptiklängsende (12) in dem Bereich des Optikkörpers (2) angeordnet ist, wobei die zweite Haptik (4) einen Beabstandungszustand, in dem das zweite Haptiklängsende (13) in einem zweiten Radialabstand (32) zu dem Optikkörper (2) in Richtung zu der optischen Achse (11) hin angeordnet ist, und einen Annäherungszustand hat, in dem das zweite Haptiklängsende (13) auf den Optikkörper (2) verlagert ist, wodurch in der Axialrichtung (16) gesehen das zweite Haptiklängsende (13) in dem Bereich des Optikkörpers (2) angeordnet ist, **dadurch gekennzeichnet, dass** die erste Haptik (3) ein erstes magnetisierbares Bauteil aufweist, die zweite Haptik (4) ein zweites magnetisierbares Bauteil aufweist, der Injektor (21) einen ersten Elektromagneten (25) aufweist, der eingerichtet ist, die erste Haptik (3) von dem Beabstandungszustand in den Annäherungszustand zu verlagern, und das Verfahren die Schritte aufweist:
- Verlagern der ersten Haptik (3) durch ein Beaufschlagen des ersten magnetisierbaren Bauteils mit einem ersten Magnetfeld von dem Beabstandungszustand in den Annäherungszustand;
- Verlagern der zweiten Haptik (4) durch ein Beaufschlagen des zweiten magnetisierbaren Bauteils mit einem zweiten Magnetfeld von dem Beabstandungszustand in den Annäherungszustand.

7. Verfahren gemäß Anspruch 6, wobei die erste Haptik (3) eine erste Pore aufweist, in die das erste Bauteil in Form von Nanopartikeln eingebracht ist und/oder die zweite Haptik (4) eine zweite Pore aufweist, in die das zweite Bauteil in Form von Nanopartikeln eingebracht ist.

8. Injektor mit einer Intraokularlinse (1), die einen Optikkörper (2), der eine optische Achse (11) aufweist, eine erste Haptik (3), die gekrümmt ausgeführt ist und ein erstes Haptiklängsende (12) aufweist, das dem Optikkörper (2) abgewandt angeordnet ist, und eine zweite Haptik (4) aufweist, die gekrümmt ausgeführt ist und ein zweites Haptiklängsende (13) aufweist, das dem Optikkörper (2) abgewandt angeordnet ist, wobei die erste Haptik (3) einen Beabstandungszustand, in dem das erste Haptiklängsende (12) in einem ersten Radialabstand (31) zu dem Optikkörper (2) in Richtung zu der optischen Achse (11) hin angeordnet ist, und einen Annäherungszustand hat, in dem das erste Haptiklängsende (12) auf den Optikkörper (2) verlagert ist, wodurch in einer Axialrichtung (16) bezüglich der optischen Achse (11) gesehen das erste Haptiklängsende (12) in dem Bereich des Optikkörpers (2) angeordnet ist, wobei die zweite Haptik (4) einen Beabstandungszustand, in dem das zweite Haptiklängsende (13) in einem zweiten Radialabstand (32) zu dem Optikkörper (2) in Richtung zu der optischen Achse (11) hin angeordnet ist, und einen Annäherungszustand hat, in dem das zweite Haptiklängsende (13) auf den Optikkörper (2) verlagert ist, wodurch in der Axialrichtung (16) gesehen das zweite Haptiklängsende (13) in dem Bereich des Optikkörpers (2) angeordnet ist, wobei die Intraokularlinse (1) in dem Injektor (21) angeordnet ist, wobei der Injektor (21) eine Injektorspitze, eine Injektoröffnung (22), einen Faltkeil, der eingerichtet ist, via die Injektoröffnung (22) innerhalb des Injektors (21) zu gelangen und dabei die Intraokularlinse (1) zu falten, eine Injektorlängsachse (27) und einen in Richtung der Injektorlängsachse (27) verlagerbaren Kolben aufweist, der eingerichtet die Intraokularlinse (1) via die Injektorspitze aus dem Injektor (21) heraus zu verlagern, **dadurch gekennzeichnet, dass** die erste Haptik (3) einen ersten Permanentmagneten (5) aufweist, die zweite Haptik (4) einen zweiten Permanentmagneten (6) aufweist, die erste Haptik (3) durch ein Beaufschlagen des ersten Permanentmagneten (5) mit einem ersten Magnetfeld von dem Beabstandungszustand in den Annäherungszustand verlagerbar ist, die zweite Haptik (4) durch ein Beaufschlagen des zweiten Permanentmagneten (6) mit einem zweiten Magnetfeld von dem Beabstandungszustand in den Annäherungszustand verlagerbar ist und der Injektor (21) einen ersten Elektromagneten (25) aufweist, der eingerichtet ist, die erste Haptik (3) von dem Beabstandungszustand in den Annäherungszustand zu verlagern.

9. Injektor mit einer Intraokularlinse (1), die einen Optikkörper (2), der eine optische Achse (11) aufweist, eine erste Haptik (3), die gekrümmt ausgeführt ist und ein erstes Haptiklängsende (12) aufweist, das dem Optikkörper (2) abgewandt angeordnet ist, und eine zweite Haptik (4) aufweist, die gekrümmt ausgeführt ist und ein zweites Haptiklängsende (13) aufweist, das dem Optikkörper (2) abgewandt angeordnet ist, wobei die erste Haptik (3) einen Beabstandungszustand, in dem das erste Haptiklängsende (12) in einem ersten Radialabstand (31) zu dem Optikkörper (2) in Richtung zu der optischen Achse (11) hin angeordnet ist, und einen Annäherungszustand hat, in dem das erste Haptiklängsende (12) auf den Optikkörper (2) verlagert ist, wodurch in einer Axialrichtung (16) bezüglich der optischen Achse (11) gesehen das erste Haptiklängsende (12) in dem Bereich des Optikkörpers (2) angeordnet ist, wobei die zweite Haptik (4) einen Beabstandungszustand, in dem das zweite Haptiklängsende (13) in einem zweiten Radialabstand (32) zu dem Optikkörper (2) in Richtung zu der optischen Achse (11) hin angeordnet ist, und einen Annäherungszustand hat, in dem das zweite Haptiklängsende (13) auf den Optikkörper (2) verlagert ist, wodurch in der Axialrichtung (16) gesehen das zweite Haptiklängsende (13) in dem Bereich des Optikkörpers (2) angeordnet ist, wobei die Intraokularlinse (1) in dem Injektor (21) angeordnet ist, wobei der Injektor (21) eine Injektorspitze, eine Injektoröffnung (22), einen Faltkeil, der eingerichtet ist, via die Injektoröffnung (22) innerhalb des Injektors (21) zu gelangen und dabei die Intraokularlinse (1) zu falten, eine Injektorlängsachse (27) und einen in Richtung der Injektorlängsachse (27) verlagerbaren Kolben aufweist, der eingerichtet die Intraokularlinse (1) via die Injektorspitze aus dem Injektor (21) heraus zu verlagern **dadurch gekennzeichnet, dass** die erste Haptik (3) ein erstes magnetisierbares Bauteil aufweist, die zweite Haptik (4) ein zweites magnetisierbares Bauteil aufweist, die erste Haptik (3) durch ein Beaufschlagen des ersten magnetisierbaren Bauteils mit einem ersten Magnetfeld von dem Beabstandungszustand in den Annäherungszustand verlagerbar ist, die zweite Haptik (4) durch ein Beaufschlagen des zweiten magnetisierbaren Bauteils mit einem zweiten Magnetfeld von dem Beabstandungszustand in den Annäherungszustand verlagerbar ist und der Injektor (21) einen ersten Elektromagneten (25) aufweist, der eingerichtet ist, die erste Haptik (3) von dem Beabstandungszustand in den Annäherungszustand zu verlagern.

10. Injektor gemäß Anspruch 8 oder 9, wobei der Injektor (21) einen ersten Führungskanal (23) und einen dem ersten Führungskanal (23) zugewandt angeordneten zweiten Führungskanal (24) aufweist, wobei in dem ersten Führungskanal (23) ein Teil des Optikkörpers (2), das Längsende (12) der ersten Haptik (3) und der erste Elektromagnet (25) angeordnet sind und in dem zweiten Führungskanal (24) ein Teil des Optikkörpers (2) und das Längsende (13) der zweiten Haptik (4) angeordnet sind.

## Claims

1. Method for displacing haptics of an intraocular lens, in which the intraocular lens (1) is arranged in an injector (21), wherein the intraocular lens (1) has an optic (2), which has an optical axis (11), a first haptic (3), which is curved and has a first haptic longitudinal end (12), which is arranged remote from the optic (2), and a second haptic (4), which is curved and has a second haptic longitudinal end (13), which is arranged remote from the optic (2), wherein the first haptic (3) has a spacing state, in which the first haptic longitudinal end (12) is arranged at a first radial distance (31) from the optic (2) in the direction of the optical axis (11), and a proximity state, in which the first haptic longitudinal end (12) has been displaced towards the optic (2), whereby the first haptic longitudinal end (12) is arranged in the region of the optic (2) when viewed in an axial direction (16) with respect to the optical axis (11), wherein the second haptic (4) has a spacing state, in which the second haptic longitudinal end (13) is arranged at a second radial distance (32) from the optic (2) in the direction of the optical axis (11), and a proximity state, in which the second haptic longitudinal end (13) has been displaced towards the optic (2), whereby the second haptic longitudinal end (13) is arranged in the region of the optic (2) when viewed in the axial direction (16), **characterized in that** the first haptic (3) has a first permanent magnet (5), the second haptic (4) has a second permanent magnet (6), the injector (21) has a first electromagnet (25), which is designed to displace the first haptic (3) from the spacing state into the proximity state, and the method comprises the steps of:
- displacing the first haptic (3) from the spacing state into the proximity state by applying a first magnetic field to the first permanent magnet (5);
- displacing the second haptic (4) from the spacing state into the proximity state by applying a second magnetic field to the second permanent magnet (6).

2. Method according to Claim 1, wherein the first permanent magnet (5) is arranged in a haptic region of the first haptic (3) and extends from the first haptic longitudinal end (12) up to a maximum of 50%, in particular a maximum of 20% or a maximum of 10%, of the overall length of the first haptic (3) and/or wherein the second permanent magnet (6) is arranged in a haptic region of the second haptic (4) that extends from the second haptic longitudinal end (13) up to a maximum of 50%, in particular a maximum of 20% or a maximum of 10%, of the overall length of the second haptic (4).

3. Method according to Claim 1 or 2, wherein a midpoint (29) between a north pole (9) and a south pole (10) of the first permanent magnet (5) extends substantially along the longitudinal direction of the first haptic (3) and a midpoint (30) between a north pole (9) and/or a south pole (10) of the second permanent magnet (6) extends substantially along a longitudinal direction of the second haptic (4).

4. Method according to one of Claims 1 to 3, wherein the first permanent magnet (5) takes the form of a first coating applied to the first haptic (3) and/or the second permanent magnet (6) takes the form of a second coating applied to the second haptic (4).

5. Method according to one of Claims 1 to 4, wherein the first haptic (3) has a first enclosure (16), which is non-magnetic and non-magnetizable and completely encloses the first permanent magnet (5), and/or wherein the second haptic (4) has a second enclosure (17), which is non-magnetic and non-magnetizable and completely encloses the second permanent magnet (6).

6. Method for displacing haptics of an intraocular lens, in which the intraocular lens (1) is arranged in an injector (21), wherein the intraocular lens (1) has an optic (2), which has an optical axis (11), a first haptic (3), which is curved and has a first haptic longitudinal end (12), which is arranged remote from the optic (2), and a second haptic (4), which is curved and has a second haptic longitudinal end (13), which is arranged remote from the optic (2), wherein the first haptic (3) has a spacing state, in which the first haptic longitudinal end (12) is arranged at a first radial distance (31) from the optic (2) in the direction of the optical axis (11), and a proximity state, in which the first haptic longitudinal end (12) has been displaced towards the optic (2), whereby the first haptic longitudinal end (12) is arranged in the region of the optic (2) when viewed in an axial direction (16) with respect to the optical axis (11), wherein the second haptic (4) has a spacing state, in which the second haptic longitudinal end (13) is arranged at a second radial distance (32) from the optic (2) in the direction of the optical axis (11), and a proximity state, in which the second haptic longitudinal end (13) has been displaced towards the optic (2), whereby the second haptic longitudinal end (13) is arranged in the region of the optic (2) when viewed in the axial direction (16), **characterized in that** the first haptic (3) has a first magnetizable component, the second haptic (4) has a second magnetizable component, the injector (21) has a first electromagnet (25), which is designed to displace the first haptic (3) from the spacing state into the proximity state, and the method comprises the steps of:
- displacing the first haptic (3) from the spacing state into the proximity state by applying a first magnetic field to the first magnetizable component;
- displacing the second haptic (4) from the spacing state into the proximity state by applying a second magnetic field to the second magnetizable component.

7. Method according to Claim 6, wherein the first haptic (3) has a first pore, in which the first component is incorporated in the form of nanoparticles, and/or the second haptic (4) has a second pore, in which the second component is incorporated in the form of nanoparticles.

8. Injector with an intraocular lens (1), which has an optic (2), which has an optical axis (11), a first haptic (3), which is curved and has a first haptic longitudinal end (12), which is arranged remote from the optic (2), and a second haptic (4), which is curved and has a second haptic longitudinal end (13), which is arranged remote from the optic (2), wherein the first haptic (3) has a spacing state, in which the first haptic longitudinal end (12) is arranged at a first radial distance (31) from the optic (2) in the direction of the optical axis (11), and a proximity state, in which the first haptic longitudinal end (12) has been displaced towards the optic (2), whereby the first haptic longitudinal end (12) is arranged in the region of the optic (2) when viewed in an axial direction (16) with respect to the optical axis (11), wherein the second haptic (4) has a spacing state, in which the second haptic longitudinal end (13) is arranged at a second radial distance (32) from the optic (2) in the direction of the optical axis (11), and a proximity state, in which the second haptic longitudinal end (13) has been displaced towards the optic (2), whereby the second haptic longitudinal end (13) is arranged in the region of the optic (2) when viewed in the axial direction (16), wherein the intraocular lens (1) is arranged in the injector (21), wherein the injector (21) has an injector tip, an injector opening (22), a folding wedge, which is designed to get inside the injector (21) via the injector opening (22) and thereby fold the intraocular lens (1), an injector longitudinal axis (27) and a plunger, which is displaceable in the direction of the injector longitudinal axis (27) and is designed to displace the intraocular lens (1) out of the injector (21) via the injector tip, **characterized in that** the first haptic (3) has a first permanent magnet (5), the second haptic (4) has a second permanent magnet (6), the first haptic (3) is displaceable from the spacing state into the proximity state by applying a first magnetic field to the first permanent magnet (5), the second haptic (4) is displaceable from the spacing state into the proximity state by applying a second magnetic field to the second permanent magnet (6) and the injector (21) has a first electromagnet (25), which is designed to displace the first haptic (3) from the spacing state into the proximity state.

9. Injector with an intraocular lens (1), which has an optic (2), which has an optical axis (11), a first haptic (3), which is curved and has a first haptic longitudinal end (12), which is arranged remote from the optic (2), and a second haptic (4), which is curved and has a second haptic longitudinal end (13), which is arranged remote from the optic (2), wherein the first haptic (3) has a spacing state, in which the first haptic longitudinal end (12) is arranged at a first radial distance (31) from the optic (2) in the direction of the optical axis (11), and a proximity state, in which the first haptic longitudinal end (12) has been displaced towards the optic (2), whereby the first haptic longitudinal end (12) is arranged in the region of the optic (2) when viewed in an axial direction (16) with respect to the optical axis (11), wherein the second haptic (4) has a spacing state, in which the second haptic longitudinal end (13) is arranged at a second radial distance (32) from the optic (2) in the direction of the optical axis (11), and a proximity state, in which the second haptic longitudinal end (13) has been displaced towards the optic (2), whereby the second haptic longitudinal end (13) is arranged in the region of the optic (2) when viewed in the axial direction (16), wherein the intraocular lens (1) is arranged in the injector (21), wherein the injector (21) has an injector tip, an injector opening (22), a folding wedge, which is designed to get inside the injector (21) via the injector opening (22) and thereby fold the intraocular lens (1), an injector longitudinal axis (27) and a plunger, which is displaceable in the direction of the injector longitudinal axis (27) and is designed to displace the intraocular lens (1) out of the injector (21) via the injector tip, **characterized in that** the first haptic (3) has a first magnetizable component, the second haptic (4) has a second magnetizable component, the first haptic (3) is displaceable from the spacing state into the proximity state by applying a first magnetic field to the first magnetizable component, the second haptic (4) is displaceable from the spacing state into the proximity state by applying a second magnetic field to the second magnetizable component and the injector (21) has a first electromagnet (25), which is designed to displace the first haptic (3) from the spacing state into the proximity state.

10. Injector according to Claim 8 or 9, wherein the injector (21) has a first guiding channel (23) and a second guiding channel (24), arranged facing the first guiding channel (23), wherein part of the optic (2), the longitudinal end (12) of the first haptic (3) and the first electromagnet (25) are arranged in the first guiding channel (23) and part of the optic (2) and the longitudinal end (13) of the second haptic (4) are arranged in the second guiding channel (24).

## Revendications

1. Procédé de déplacement d'haptiques d'une lentille intraoculaire, selon lequel la lentille intraoculaire (1) est disposée dans un injecteur (21), la lentille intraoculaire (1) comprenant un corps optique (2) qui présente un axe optique (11), une première haptique (3) qui est réalisée de manière incurvée et présente une première extrémité longitudinale d'haptique (12) qui est disposée à l'opposé du corps optique (2), et une deuxième haptique (4) qui est réalisée de manière incurvée et présente une deuxième extrémité longitudinale d'haptique (13) qui est disposée à l'opposé du corps optique (2), la première haptique (3) présentant un état d'espacement dans lequel la première extrémité longitudinale d'haptique (12) est disposée à une première distance radiale (31) par rapport au corps optique (2) en direction de l'axe optique (11), et un état de rapprochement dans lequel la première extrémité longitudinale d'haptique (12) est déplacée sur le corps optique (2), de sorte que, vue dans une direction axiale (16) par rapport à l'axe optique (11), la première extrémité longitudinale d'haptique (12) est disposée dans la zone du corps optique (2), la deuxième haptique (4) présentant un état d'espacement dans lequel la deuxième extrémité longitudinale d'haptique (13) est disposée à une deuxième distance radiale (32) par rapport au corps optique (2) en direction de l'axe optique (11), et un état de rapprochement dans lequel la deuxième extrémité longitudinale d'haptique (13) est déplacée sur le corps optique (2), de sorte que, vue dans la direction axiale (16), la deuxième extrémité longitudinale d'haptique (13) est disposée dans la zone du corps optique (2), **caractérisé en ce que** la première haptique (3) comporte un premier aimant permanent (5), la deuxième haptique (4) comporte un deuxième aimant permanent (6), l'injecteur (21) comporte un premier électroaimant (25) qui est conçu pour déplacer la première haptique (3) de l'état d'espacement à l'état de rapprochement, et le procédé comprend les étapes suivantes :
- déplacement de la première haptique (3) de l'état d'espacement à l'état de rapprochement par application d'un premier champ magnétique au premier aimant permanent (5) ;
- déplacement de la deuxième haptique (4) de l'état d'espacement à l'état de rapprochement par application d'un deuxième champ magnétique au deuxième aimant permanent (6).

2. Procédé selon la revendication 1, dans lequel le premier aimant permanent (5) est disposé dans une zone haptique de la première haptique (3) qui s'étend de la première extrémité longitudinale d'haptique (12) jusqu'à un maximum de 50 %, en particulier un maximum de 20 % ou un maximum de 10 %, de la longueur totale de la première haptique (3), et/ou dans lequel le deuxième aimant permanent (6) est disposé dans une zone haptique de la deuxième haptique (4) qui s'étend de la deuxième extrémité longitudinale d'haptique (13) jusqu'à un maximum de 50 %, en particulier un maximum de 20 % ou un maximum de 10 %, de la longueur totale de la deuxième haptique (4).

3. Procédé selon la revendication 1 ou 2, dans lequel un centre (29) situé entre un pôle nord (9) et un pôle sud (10) du premier aimant permanent (5) s'étend sensiblement le long d'une direction longitudinale de la première haptique (3), et un centre (30) situé entre un pôle nord (9) et/ou un pôle sud (10) du deuxième aimant permanent (6) s'étend sensiblement le long d'une direction longitudinale de la deuxième haptique (4).

4. Procédé selon l'une des revendications 1 à 3, dans lequel le premier aimant permanent (5) est monté sur la première haptique (3) en tant que premier revêtement, et/ou dans lequel le deuxième aimant permanent (6) est monté sur la deuxième haptique (4) en tant que deuxième revêtement.

5. Procédé selon l'une des revendications 1 à 4, dans lequel la première haptique (3) présente un premier gainage (16), qui est non magnétique et non magnétisable et revêt complètement le premier aimant permanent (5), et/ou dans lequel la deuxième haptique (4) présente un deuxième gainage (17), qui est non magnétique et non magnétisable et revêt complètement le deuxième aimant permanent (6).

6. Procédé de déplacement d'haptiques d'une lentille intraoculaire, selon lequel la lentille intraoculaire (1) est disposée dans un injecteur (21), la lentille intraoculaire (1) comprenant un corps optique (2) qui présente un axe optique (11), une première haptique (3) qui est réalisée de manière incurvée et présente une première extrémité longitudinale d'haptique (12) qui est disposée à l'opposé du corps optique (2), et une deuxième haptique (4) qui est réalisée de manière incurvée et présente une deuxième extrémité longitudinale d'haptique (13) qui est disposée à l'opposé du corps optique (2), la première haptique (3) présentant un état d'espacement dans lequel la première extrémité longitudinale d'haptique (12) est disposée à une première distance radiale (31) par rapport au corps optique (2) en direction de l'axe optique (11), et un état de rapprochement dans lequel la première extrémité longitudinale d'haptique (12) est déplacée sur le corps optique (2), de sorte que, vue dans une direction axiale (16) par rapport à l'axe optique (11), la première extrémité longitudinale d'haptique (12) est disposée dans la zone du corps optique (2), la deuxième haptique (4) présentant un état d'espacement dans lequel la deuxième extrémité longitudinale d'haptique (13) est disposée à une deuxième distance radiale (32) par rapport au corps optique (2) en direction de l'axe optique (11), et un état de rapprochement dans lequel la deuxième extrémité longitudinale d'haptique (13) est déplacée sur le corps optique (2), de sorte que, vue dans la direction axiale (16), la deuxième extrémité longitudinale d'haptique (13) est disposée dans la zone du corps optique (2), **caractérisé en ce que** la première haptique (3) comporte un premier composant magnétisable, la deuxième haptique (4) comporte un deuxième composant magnétisable, l'injecteur (21) comporte un premier électroaimant (25) qui est conçu pour déplacer la première haptique (3) de l'état d'espacement à l'état de rapprochement, et le procédé comprend les étapes suivantes :
- déplacement de la première haptique (3) de l'état d'espacement à l'état de rapprochement par application d'un premier champ magnétique au premier composant magnétisable ;
- déplacement de la deuxième haptique (4) de l'état d'espacement à l'état de rapprochement par application d'un deuxième champ magnétique au deuxième composant magnétisable.

7. Procédé selon la revendication 6, dans lequel la première haptique (3) présente un premier pore dans lequel le premier composant est introduit sous forme de nanoparticules, et/ou dans lequel la deuxième haptique (4) présente un deuxième pore dans lequel le deuxième composant est introduit sous forme de nanoparticules.

8. Injecteur doté d'une lentille intraoculaire (1) comprenant un corps optique (2) qui présente un axe optique (11), une première haptique (3) qui est réalisée de manière incurvée et présente une première extrémité longitudinale d'haptique (12) qui est disposée à l'opposé du corps optique (2), et une deuxième haptique (4) qui est réalisée de manière incurvée et présente une deuxième extrémité longitudinale d'haptique (13) qui est disposée à l'opposé du corps optique (2), la première haptique (3) présentant un état d'espacement dans lequel la première extrémité longitudinale d'haptique (12) est disposée à une première distance radiale (31) par rapport au corps optique (2) en direction de l'axe optique (11), et un état de rapprochement dans lequel la première extrémité longitudinale d'haptique (12) est déplacée sur le corps optique (2), de sorte que, vue dans une direction axiale (16) par rapport à l'axe optique (11), la première extrémité longitudinale d'haptique (12) est disposée dans la zone du corps optique (2), la deuxième haptique (4) présentant un état d'espacement dans lequel la deuxième extrémité longitudinale d'haptique (13) est disposée à une deuxième distance radiale (32) par rapport au corps optique (2) en direction de l'axe optique (11), et un état de rapprochement dans lequel la deuxième extrémité longitudinale d'haptique (13) est déplacée sur le corps optique (2), de sorte que, vue dans la direction axiale (16), la deuxième extrémité longitudinale d'haptique (13) est disposée dans la zone du corps optique (2), la lentille intraoculaire (1) étant disposée dans l'injecteur (21), l'injecteur (21) présentant une pointe d'injecteur, une ouverture d'injecteur (22), un coin de pliage qui est conçu pour pénétrer dans l'injecteur (21) via l'ouverture d'injecteur (22) et plier ainsi la lentille intraoculaire (1), un axe longitudinal d'injecteur (27) et un piston qui est déplaçable dans la direction de l'axe longitudinal d'injecteur (27) et est conçu pour déplacer la lentille intraoculaire (1) hors de l'injecteur (21) via la pointe d'injecteur, **caractérisé en ce que** la première haptique (3) comporte un premier aimant permanent (5), la deuxième haptique (4) comporte un deuxième aimant permanent (6), la première haptique (3) est déplaçable de l'état d'espacement à l'état de rapprochement par application d'un premier champ magnétique au premier aimant permanent (5), la deuxième haptique (4) est déplaçable de l'état d'espacement à l'état de rapprochement par application d'un deuxième champ magnétique au deuxième aimant permanent (6) et l'injecteur (21) comporte un premier électroaimant (25) qui est conçu pour déplacer la première haptique (3) de l'état d'espacement à l'état de rapprochement.

9. Injecteur doté d'une lentille intraoculaire (1) comprenant un corps optique (2) qui présente un axe optique (11), une première haptique (3) qui est réalisée de manière incurvée et présente une première extrémité longitudinale d'haptique (12) qui est disposée à l'opposé du corps optique (2), et une deuxième haptique (4) qui est réalisée de manière incurvée et présente une deuxième extrémité longitudinale d'haptique (13) qui est disposée à l'opposé du corps optique (2), la première haptique (3) présentant un état d'espacement dans lequel la première extrémité longitudinale d'haptique (12) est disposée à une première distance radiale (31) par rapport au corps optique (2) en direction de l'axe optique (11), et un état de rapprochement dans lequel la première extrémité longitudinale d'haptique (12) est déplacée sur le corps optique (2), de sorte que, vue dans une direction axiale (16) par rapport à l'axe optique (11), la première extrémité longitudinale d'haptique (12) est disposée dans la zone du corps optique (2), la deuxième haptique (4) présentant un état d'espacement dans lequel la deuxième extrémité longitudinale d'haptique (13) est disposée à une deuxième distance radiale (32) par rapport au corps optique (2) en direction de l'axe optique (11), et un état de rapprochement dans lequel la deuxième extrémité longitudinale d'haptique (13) est déplacée sur le corps optique (2), de sorte que, vue dans la direction axiale (16), la deuxième extrémité longitudinale d'haptique (13) est disposée dans la zone du corps optique (2), la lentille intraoculaire (1) étant disposée dans l'injecteur (21), l'injecteur (21) présentant une pointe d'injecteur, une ouverture d'injecteur (22), un coin de pliage qui est conçu pour pénétrer dans l'injecteur (21) via l'ouverture d'injecteur (22) et plier ainsi la lentille intraoculaire (1), un axe longitudinal d'injecteur (27) et un piston qui est déplaçable dans la direction de l'axe longitudinal d'injecteur (27) et est conçu pour déplacer la lentille intraoculaire (1) hors de l'injecteur (21) via la pointe d'injecteur, **caractérisé en ce que** la première haptique (3) comporte un premier composant magnétisable, la deuxième haptique (4) comporte un deuxième composant magnétisable, la première haptique (3) est déplaçable de l'état d'espacement à l'état de rapprochement par application d'un premier champ magnétique au premier composant magnétisable, la deuxième haptique (4) est déplaçable de l'état d'espacement à l'état de rapprochement par application d'un deuxième champ magnétique au deuxième composant magnétisable et l'injecteur (21) comporte un premier électroaimant (25) qui est conçu pour déplacer la première haptique (3) de l'état d'espacement à l'état de rapprochement.

10. Injecteur selon la revendication 8 ou 9, dans lequel l'injecteur (21) présente un premier canal de guidage (23) et un deuxième canal de guidage (24) disposé en face du premier canal de guidage (23), une partie du corps optique (2), l'extrémité longitudinale (12) de la première haptique (3) et le premier électroaimant (25) étant disposés dans le premier canal de guidage (23), et une partie du corps optique (2) et l'extrémité longitudinale (13) de la deuxième haptique (4) étant disposées dans le deuxième canal de guidage (24).
